# EUROPEAN PATENT SPECIFICATION

(11) **EP 0 884 601 B1**
(45) Date of publication and mention of the grant of the patent: **02.10.2002**
(21) Application number: 97304085.0
(22) Date of filing: 11.06.1997
(51) Int. Cl.: G01R 33/035, A61B 5/04

(54) **Magnetometer**
Magnetometer
Magnétomètre

(43) Date of publication of application: 16.12.1998
(73) Proprietor: Kanazawa Institute of Technology, Ishigawa-gun, Ishikawa (JP)
(72) Inventor: Kado, Hisashi, Kashiwa-shi, Chiba (JP)
(74) Representative: SERJEANTS

(56) References cited:
- EP-A- 0 200 958
- EP-A- 0 361 137
- EP-A- 0 492 262
- EP-A- 0 492 263
- EP-A- 0 595 227

## Description

### BACKGROUND OF THE INVENTION

The present invention relates to an apparatus for measuring weak magnetic fields using superconducting quantum interference devices (SQUIDs) as magnetic field sensors, a method for assembling the same and a method for maintaining the same, and a diagnostic apparatus using the same.

An apparatus for measuring weak magnetic fields using SQUIDs as magnetic field sensors is known. The SQUIDs have so high sensitivities that the measuring apparatus using the SQUIDs can measure weak biomagnetic fields.

As one of such measuring apparatuses, a weak magnetic field measuring apparatus for measuring magnetic fields generated in, e.g., a head of a human being is known (see Japanese Patent Laid-Open Publication No. Tokkai Hei 04-315075/1992).

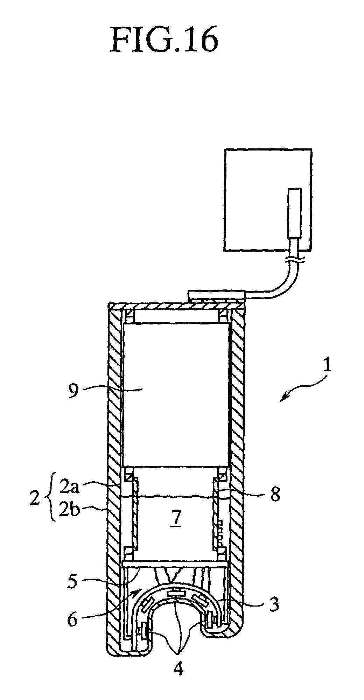

As shown in FIG. 16, the weak magnetic field measuring device 1 comprises a Dewar vessel 2 including an inner vessel 2a and an outer vessel 2b and the bottom of the Dewar vessel 2 shaped in conformity with a head of a human being. A support shell 3 is accommodated in the bottom of the Dewar vessel 2. The support shell 3 is formed in a shape which is contour to a shape of the bottom of the Dewar vessel 2. A plurality of SQUIDs 4 are arranged in the support shell 3, enclosing the bottom. The support shell 3 constitutes one unit 6 together with a mother board 5 in the form of a printed circuit board, etc. This unit 6 is submerged in liquid helium, a coolant. A neck plug 9 is disposed above the unit 6 through a support 8 and is housed in an upper part of the interior of the Dewar vessel 2. The neck plug 9 comprises a radiation shielding and cabling unit. The support 8 has electric components mounted thereon.

The respective component members in the Dewar vessel 2 are provided in units, and the unit 6 of the support shell 3 with the neck plug 9 and the support 8 can be removed from the Dewar vessel 2 independently.

In order that such a measuring apparatus measures, with precision, a magnetic field over an extended area, it is necessary to arrange a number of magnetic field sensors over that extended area. Accordingly, the SQUIDs 4 of the weak magnetic field measuring apparatus 1 are mounted on the support shell 3 formed in a helmet-shape which can cover a head over a wide area. The support shell 3 must have a diameter of at least 30 cm, and the opening of the Dewar vessel 2 through which the unit 6, including the support shell 3, is inserted similarly must have a diameter above 30 cm.

Liquid helium, a coolant, is very volatile, and it is necessary to minimize intrusion of heat from the outside. Major routes of the heat intrusion into the Dewar vessel 2 are the vacuum heat-insulating layer between the inner vessel 2a and the outer vessel 2b, and the opening of the Dewar vessel 2. Loss of the liquid helium due to heat conduction in the opening and the wall of the Dewar vessel 2a occupies a considerably high ratio, and the intruding heat reaches a considerably large total amount.

Thus, a large diameter of the opening of the Dewar vessel 2 increases loss of the liquid helium in the Dewar vessel 2 due to the intruded heat. Expensive liquid helium must be frequently supplied, which results in a disadvantage of higher maintenance costs.

An increase in the length of a neck opening of the Dewar vessel 2 reduces the heat loss by the heat conduction, but the Dewar vessel 2 becomes taller. The taller Dewar vessel 2 thus requires a taller room.

An object of the present invention is to provide a measuring apparatus for measuring weak magnetic fields, the vessel of which has a small opening so that the apparatus has optimum thermal insulation; and a method for assembling the same; and a method for maintaining the same.

Another object of the present invention is to provide a diagnostic apparatus using such a measuring apparatus.

According to the invention an apparatus for measuring weak biomagnetic fields including a plurality of magnetic field sensors and a coolant vessel having an interior with a measuring surface, said measuring surface being an interior surface of said coolant vessel, and a neck opening is provided, comprising: a sensor support for disengageably supporting said magnetic field sensors, said support being disposed in said interior at said measuring surface; and fixing means for disengageably fixing the sensor support, with said plural magnetic field sensors mounted thereon, to said measuring surface of the coolant vessel, the disengageability of the fixing means permitting disengagement of the magnetic field sensors from the measuring surface; wherein the opening of the coolant vessel provides access for the magnetic field sensors and has a diameter smaller than that of the sensor support but larger than that of the magnetic field sensors to permit installation of the magnetic field sensors on said sensor support via said opening.

The apparatus of the invention is particularly suited for use as magnetoencephalographic apparatus. For example, the measuring surface of the coolant vessel may be formed in a side of the coolant vessel which follows the shape of a concavity in an outside of the coolant vessel, which concavity is adapted to cover at least a part of a head of a human being; and the sensor support may have a configuration which permits the magnetic field sensors to be disposed in conformance with a contour of the measuring surface.

According to another aspect of the invention an assembly method for assembling the measuring apparatus of the invention is provided, comprising the steps of disengaging the sensor support from the measuring surface of the coolant vessel, holding the sensor support within the coolant vessel near to the opening of the coolant vessel, mounting the plurality of magnetic field sensors on the sensor support, and releasably fixing the sensor support with the plurality of magnetic field sensors mounted on to the measuring surface of the coolant vessel.

According to yet another aspect of the invention a measuring apparatus maintenance method for maintaining the measuring apparatus is provided, comprising the steps of disengaging the sensor support with the plurality of magnetic field sensors from the measuring surface of the coolant vessel, holding the sensor support within the coolant vessel near to the opening of the coolant vessel, removing a defective one of the plurality of magnetic field sensors from the senor support, mounting a normal magnetic field sensor in place of the defective one, and releasably fixing the sensor support with the normal magnetic field sensor to the measuring surface of the coolant vessel.

The invention further provides a diagnostic apparatus for measuring weak magnetic fields comprising: a bed for a patient to be diagnosed to lie on; the above-described measuring apparatus disposed on an end of the bed and arranged so that the head of the patient can be positioned near to the measuring surface.

### BRIEF DESCRIPTION OF THE DRAWINGS

FIG. 1 is a perspective view of a measuring apparatus according to a first embodiment of the present invention.
FIG. 2 is a vertical section through the measuring apparatus according to the first embodiment of the present invention.
FIG. 3 is a cross-sectional view of the measuring apparatus according to the first embodiment of the present invention, which shows an arrangement of magnetic field sensors.
FIGs. 4A and 4B are views illustrating an example of a structure for mounting the magnetic field sensors on the sensor support in the measuring apparatus according to the first embodiment of the present invention.
FIG. 5 is a view illustrating the process of mounting the magnetic field sensors on the sensor support in the measuring apparatus according to the first embodiment of the present invention.
FIGs. 6A to 6C are views illustrating a method for assembling the measuring apparatus according to the first embodiment of the present invention.
FIGs. 7A to 7C are views illustrating a method for maintaining the measuring apparatus according to the first embodiment of the present invention.
FIG. 8 is a vertical section through the measuring apparatus according to the first embodiment of the present invention, which illustrates another example of means for releasably securing the sensor support to the bottom of the inner vessel body.
FIG. 9 is a vertical section through the measuring apparatus according to the first embodiment of the present invention, illustrating another example of means for lifting the sensor support.
FIG. 10 is a perspective view of a measuring apparatus according to a second embodiment of the present invention.
FIG. 11 is a vertical section through a measuring apparatus according to the second embodiment of the present invention.
FIG. 12 is a perspective view of diagnostic apparatus using the measuring apparatus according to the second embodiment of the present invention.
FIG. 13 is a plan view of the diagnostic apparatus using the measuring apparatus according to the second embodiment of the present invention.
FIG. 14 is a front view of the diagnostic apparatus using the measuring apparatus according to the second embodiment of the present invention.
FIG. 15 is a side view of the diagnostic apparatus using the measuring apparatus according to the second embodiment of the present invention.
FIG. 16 is a sectional view of a conventional measuring apparatus.

### DETAILED DESCRIPTION OF THE INVENTION

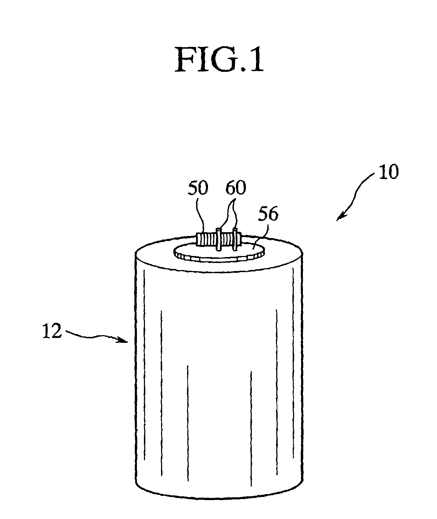

The measuring apparatus according to a first embodiment of the present invention will be explained with reference of FIGs. 1 to 9.

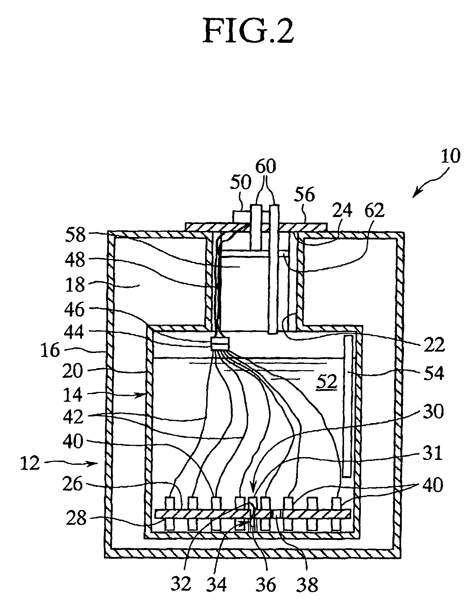

As shown in FIG. 1, the measuring apparatus 10 includes a cryostat 12 which is a bottomed cylindrical vessel. As shown in FIG. 2, the cryostat 12 has a double structure of an inner bottomed cylindrical vessel 14 accommodated in an outer bottomed cylindrical vessel 16. A heat insulating material 18 fills the space between the inner vessel 14 and the outer vessel 16.

The inner vessel 14 includes an inner vessel body 20, and a cylindrical neck opening 22 projected from a top part of the inner vessel body 20. The neck opening 22 which is an outlet has an inner diameter smaller than that of the inner vessel body 20. The neck opening 22 has an about 14 cm-inner diameter and an about 30 cm-height. The outer vessel 16 has a top opening 24 which is in communication with the neck opening 22.

A sensor support 26 is accommodated in the inner vessel body 20. The sensor support 26 is a disc shape and is a little smaller than the inner diameter of the inner vessel body 20. Its diameter is 30- 40 cm. A plurality of holes 28 are formed at a substantially constant pitch in the entire surface of the sensor support 26.

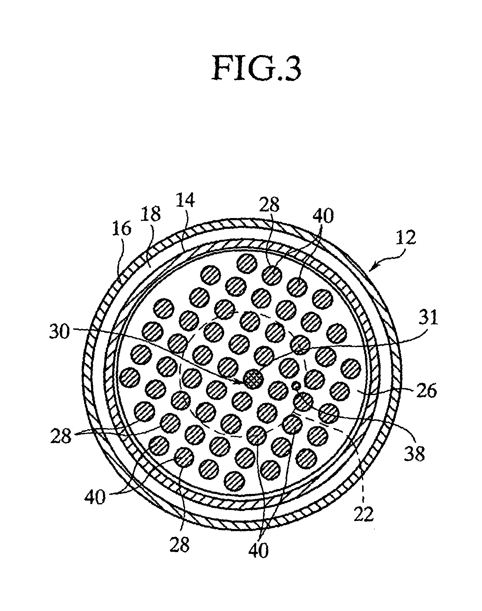

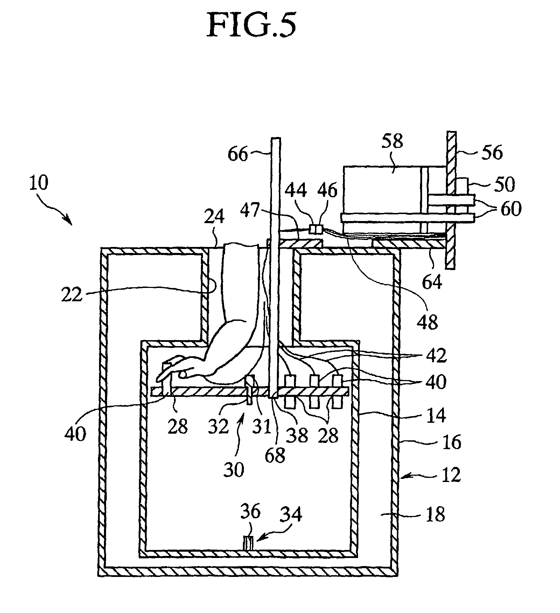

As shown in FIG. 3, a male clamp 30 is provided substantially on the center of the surface of the sensor support 26. As shown in FIGs. 2 and 5, the male clamp 30 includes a knob 31 projected to the front surface of the sensor support 26 and an insert 32 projected to the back surface of the sensor support 26.

A female clamp 34 corresponding to the male clamp 30 is provided on the bottom of the vessel body 20. The female clamp 34 includes a receiver 36 for holding the insert 32 engaged therein. The insert 32 of the male clamp 30 is pushed in the receiver 36 of the female clamp 34, whereby, as shown in FIG. 2, the insert 32 is engaged with the receiver 36 and the male clamp 30 is connected to the female clamp 34. The insert 32 of the male clamp 30 is pulled out of the receiver 36 of the female clamp 34, whereby, as shown in FIG. 5, the insert 32 is disengaged from the receiver 36, and the male clamp 30 is separated from the female clamp 34. The clamps 30 and 34 may be replaced by screws.

As shown in FIGs. 2 and 3, a tapped hole 38 is formed substantially in the center of the surface of the sensor support 26. The tapped hole 38 may be assembled with the male clamp 30. A lift rod 66, which will be described below, can be screwed into the tapped hole 38.

As shown in FIG. 3, magnetic field sensors 40 comprising SQUIDs are engaged fixedly in the respective holes 28 in the sensor support 26. A plurality of magnetic field sensors 40 are fixed in the sensor support 26 regularly at a constant pitch over substantially the entire surface of the sensor support 26. The magnetic field sensors 40 comprising the SQUIDs, which can measure weak biomagnetic fields, can measure magnetic fields generated in a head of a human being.

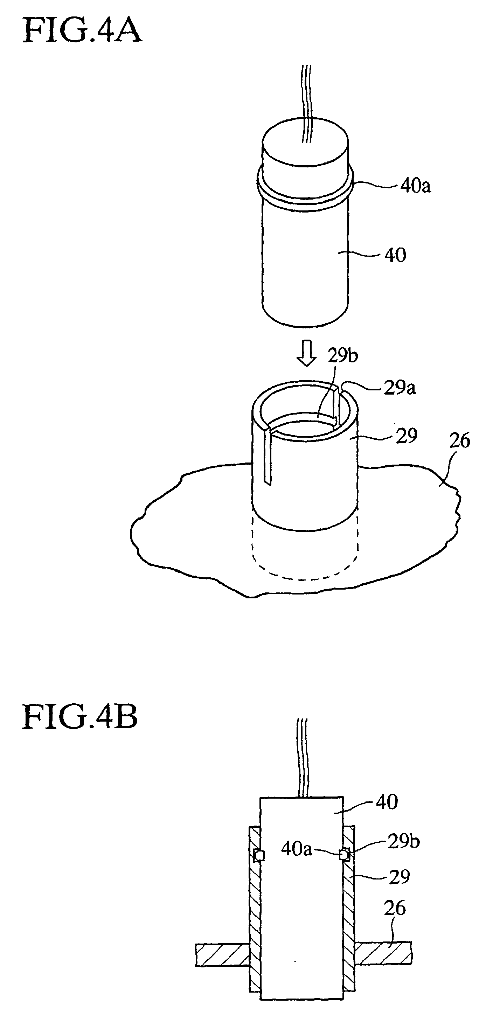

With reference to FIGs. 4A, 4B, an example of the structure for mounting the magnetic field sensors 40 on the sensor support 26 will be explained.

As shown in FIG. 4A, sensor supports 29 for supporting the magnetic field sensors 40 are provided in the respective holes 28 in the sensor support 26. Each sensor support 29 is in the shape of a thin cylinder. Slits 29a are formed in 2 to 4 upper parts of the cylinder, and a groove 29b is formed in the inside surface thereof. A ring 40a is formed on the circumference of each magnetic field sensor 40.

As shown in FIG. 4B, when a magnetic field sensor 40 is inserted into the sensor support 29, the slits 29a allow the upper part of the sensor support 29 to be a little expanded outward to facilitate the engagement of the magnetic field sensor 40. When the magnetic field sensor 40 is further engaged into the sensor support 29, the ring 40a is engaged into the groove 29b in the sensor support 29 and positioned there.

To measure a magnetic field distribution over a wide area it is necessary to arrange a number of magnetic field sensors 40 over that wide area. To this end, the sensor support 26 is formed to be sufficiently wide to accommodate a number of magnetic field sensors 40 necessary for objects to be measured.

As shown in FIG. 2, measuring wires 42 are led out from the respective magnetic field sensors 40. The respective measuring wires 42 leading from the respective magnetic field sensors 40 are collected by a wire connector 44. The wire connector 44 is connected to a plug connector 46. A wiring loom 48 leads from the plug connector 46 to an output connector 50 disposed on the outside surface of a cap 56, which will be described later, through a gap between a heat insulator 58 and the neck opening 22. The wiring loom 48 is formed of a high electric resistance wire having low heat conductivity, whereby heat intrusion due to heat conduction can be suppressed at a low level.

The inner vessel body 20 holds liquid helium 52 in which the magnetic field sensors 40 are submerged (see FIG. 2). The liquid helium is a coolant for the magnetic field sensors 40. A level sensor 54 is disposed inside the inner vessel body 20 and detects a level of the liquid helium 52 held in the inner vessel body 20.

As shown in FIGs. 1 and 2, the top opening 24 of the outer vessel 16 is closed by a cap having a larger diameter than the top opening 24. The cap 56 prevents vaporization due to contact between air and the liquid helium 52. On the underside of the cap 56 there is provided a cylindrical heat insulation portion 58 having a little smaller configuration than an inner diameter of the neck opening 22 and substantially the same height as the neck opening 22. When the cap 56 closes the top opening 24, the heat insulation portion 58 is inserted into the neck opening 22.

In the cap 56 there are provided two charge/discharge pipes 60 which extend from the outer side of the cap 56. As shown in FIG. 2, one of the charge/discharge pipes 60 has its other end in communication with a cross-bore 62 of the heat insulation portion 58 for venting helium gas from the inner vessel body 20. The other of the charge/discharge pipes 60 has its other end opened at the underside of the heat insulation portion 58. Liquid helium is fed into the inner vessel body 20 through one of the charge/discharge pipes 60 and helium gas is discharged through the other.

The inner vessel 14, the outer vessel 16, and almost all constituent members of the cryostat 12 are formed of non-magnetic and non-conducting material, such as fiber-reinforced resin or others. The heat insulation portion 58 is formed of heat insulating material comprising multi-layers of polyester resin foils with aluminium vapor deposited.

The bottoms of the inner vessel 14 and the outer vessel 16 are not essentially flat and may have arbitrary shapes as required. For example, the bottoms may be helmet-shaped.

The mounting of the magnetic field sensors 40 on the sensor support 26 is carried out, when the measuring apparatus 10 is assembled at the start, and when defective ones of the magnetic field sensors 40 are replaced by new ones for maintenance.

In order to mount the magnetic field sensors 40 on the sensor support 26 having a larger diameter than an inner diameter of the neck opening 22 of the inner vessel 14, as shown in FIG. 5, an operator raises the sensor support 26 to the lower end of the neck opening 22, and puts his hand into the inner vessel 14 through the opening 22.

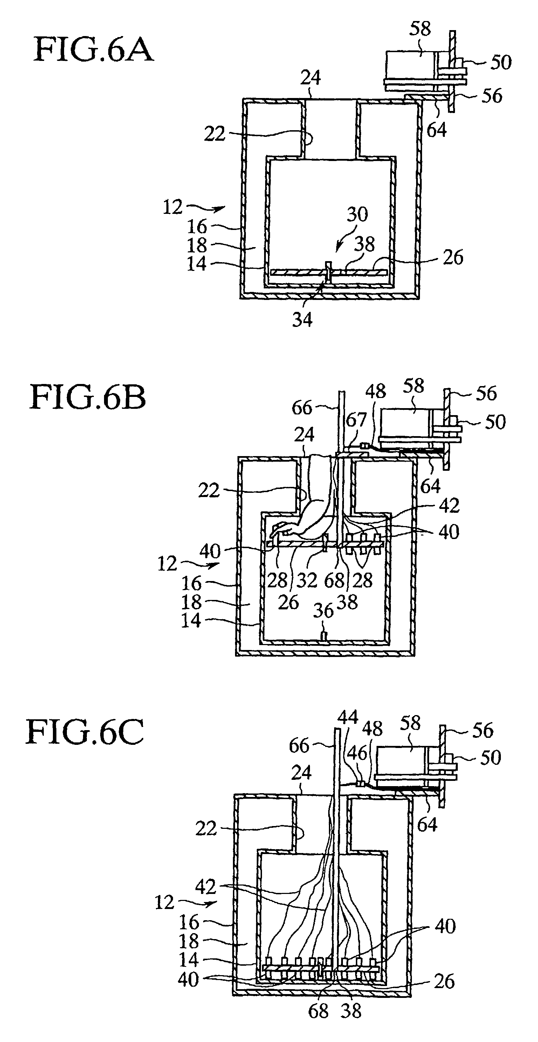

The method for assembling the measuring apparatus 10 will be explained with reference to FIG. 6.

First, the opening 24 of the outer vessel 16 of the cryostat 12 is opened. The cap 56 closing the upper opening 24 is lifted to pull out the heat insulation portion 58. The cap 56 disengaged from the top opening 24 is rested horizontally on the cap support 64. In the inner vessel 14 of the cryostat 12 the sensor support 26 is secured to the bottom surface of the inner vessel 14 by the engagement between the male clamp 30 and the female clamp 34. The sensor support 26 is beforehand accommodated in the inner vessel 14 when the inner vessel 14 is fabricated and assembled.

Then, the male clamp 30 is disengaged from the female clamp 34 by using the lift rod 66. The lift rod 66 has a length larger than a distance from the top opening 24 to the inside bottom of the inner vessel 14, and has a threaded portion 68 which can be screwed into the tapped hole 38 of the sensor support 26. The lift rod 66 is registered with the tapped hole 38 of the sensor support 26 and turned to be screw-engaged with the sensor support 26.

Subsequently, the lift rod 66 screw-engaged with the sensor support 26 is lifted. By lifting the lift rod 66, the male clamp 30 is pulled by the sensor support 26 and disengaged from the female clamp 34. After the male clamp 30 has been disengaged from the female clamp 34, the sensor support 26 may be lifted up to the vicinity of the open space 22.

Then, the lifted sensor support 26 is held in the lifted state as shown in FIG. 6B. The lift rod 66 screw-engaged in the lifted sensor support 26 is secured to the rod support 67 provided on the top surface of the outer vessel 16 over hanging the top opening 24.

Then, as shown in FIG. 6B, a magnetic field sensor 40 is inserted by hand into the neck opening 22 through the top opening 24 to be mounted on the sensor support 26. The neck opening 22 has an about 14 cm-diameter which allows the hand and arm holding a magnetic field sensor 40 to be put into the neck opening 22. The operation may be monitored through the neck opening 22 by a fiberscope, a CCD camera, a mirror or other means.

The measuring wires 42 of the respective magnetic field sensors 40 are wired to the output connector 50 through the wire connector 44 connected to the plug connector 46. The magnetic field sensors 40 are pushed into the holes 28 to be secured to the sensor support 26.

Then, as shown in FIG. 6C, the sensor support 26 with the magnetic field sensors 40 mounted thereon is secured to the bottom surface of the inner vessel 14. When the mounting of the magnetic field sensors 40 on the sensor support 26 is completed, the lift rod 66 is held by hand, released from the rod support 67 and lowered into the inner vessel 14. When the sensor support 26 comes near the bottom surface of the inner vessel 14, the male clamp 30 of the sensor support 26 is brought into register with the female clamp 34, and the lift rod 66 is pushed downward to engage the male clamp 30 with the female clamp 34.

Then, the lift rod 66 is removed from the sensor support 26 by unscrewing and lifted out of the inner vessel 14.

Thus, the magnetic field sensors 40 are mounted on the sensor support 26 without taking the sensor support 26 out of the inner vessel 14.

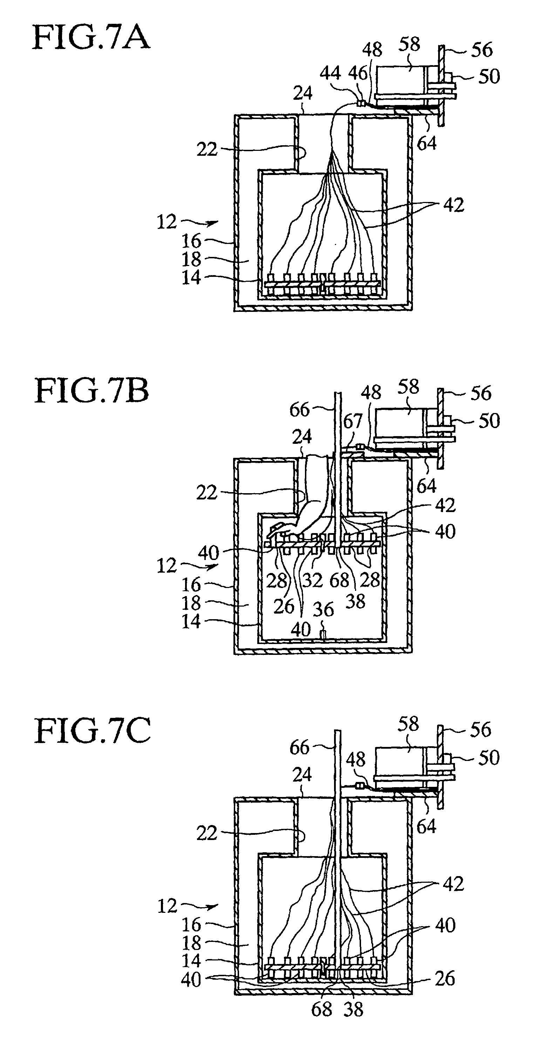

Next, the method for maintaining the measuring apparatus by replacing magnetic field sensors 40 mounted on the sensor support 26 will be explained with reference to FIG. 7. Similar steps of the maintenance method with those of the assembling method will not be explained to simplify the explanation of the maintenance method.

First, the top opening 24 of the outer vessel 16 of the cryostat 12 is opened. The cap 56 is removed from the top opening 24 and rested on the cap support 64. In the inner vessel 14 of the cryostat 12, the sensor support 26 with the magnetic field sensors 40 mounted thereon is secured to the bottom surface of the inner vessel 14. The measuring wires 42 of the respective sensors 40 are wired to the output connector 50 through wire connector 44 and plug connector 46. The liquid helium 52 in the inner vessel 14 has been discharged in advance from the inner vessel 14.

Then, the male clamp 30 is disengaged from the female clamp 34 using the lift rod 66, and the sensor support 26 is raised to a level near the neck opening 22. Then, the lifted sensor support26 is held in this state as shown in FIG. 7B.

Next, as shown in FIG. 7B, a hand is put into the neck opening 22 through the top opening 24 to pull a magnetic field sensor 40, which is to be replaced, from the sensor support 26.

The measuring wire of a new magnetic field sensor 40 replacing the pulled-out magnetic field sensor 40 is wired to the output connector 50 through the wire connector 44 connected to the plug connector 46.

Then, as shown in FIG. 7B, the new magnetic field sensor 40 whose measuring wire has been wired to the output connector 50 is held by a hand to put into the neck opening 22 through the top opening 24, and is mounted on the sensor support 26.

Then, as shown in FIG. 7C, the sensor support 26 with the new magnetic field sensor 40 mounted thereon is lowered and secured to the bottom surface of the inner vessel 14.

Then, the lift rod 66 is removed from the sensor support 26.

Thus, even if one magnetic field sensor malfunctions the defective magnetic field sensor can be replaced by a normal magnetic field sensor without taking the sensor support 26 out of the inner vessel 14.

As described above, according to the present embodiment, it suffices that the neck opening 22 has a size sufficient for a human hand to pass through, e.g., has an about 14 cm diameter, a 2 - 3 mm thickness and an about 30 cm length. The conventional measuring apparatus has a 30-40 cm diameter, a 4 - 5 mm thickness and a 40 - 50 cm length. In comparison with the conventional apparatus, the measuring apparatus according to the present invention is reduced to the diameter which is 1/2 - 1/3 of the conventional apparatus, and the length which is about 4/5 of the conventional apparatus.

The conducting heat through the wall of the neck opening 22 is proportional to the diameter of the neck opening 22, and conduction heat conducted in the heat insulation material inserted in the neck opening 22 is proportional to a sectional area, or square of a diameter of the neck opening 22, and linearly proportional to a length of the neck opening 22. Even in consideration of affection by heat conduction increase due to the smaller length, heat intrusion occurring because of the neck opening 22 can be drastically decreased in comparison with that in the conventional measuring apparatuses.

In the present embodiment, the clamps 30, 34 are used to secure the sensor support 26 to the bottom surface of the inner vessel body 20, but the sensor support 26 may be secured by different releasable means.

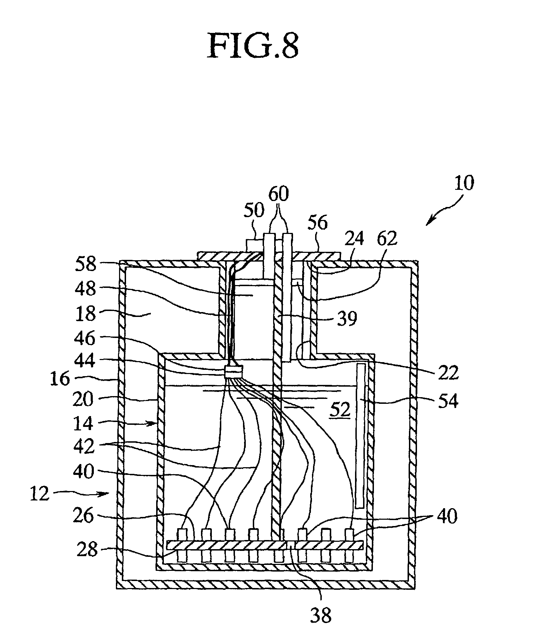

As shown in FIG. 8, for example, the sensor support 26 may be fixed by a support rod 39 which is fixed to the cap 56, and is passed through the neck opening 22 to be reached to the sensor support 26. The support rod 39 is formed of fibre-reinforced plastic. The sensor support 26 is pressed against the bottom surface of the inner vessel body 20 by flexibility exhibited when the support rod 39 is flexed. A single support rod 39 or a plurality of support rods 38 may be used.

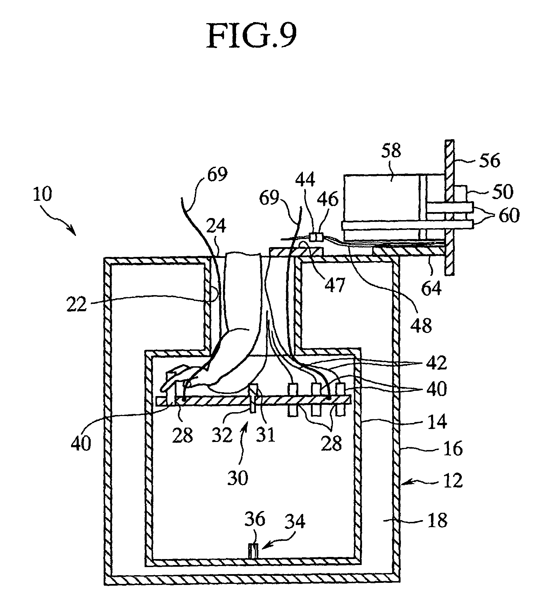

In the first embodiment the sensor support 26 is lifted by the rod 66 or 39, but it may be lifted by different means. As shown in FIG. 9, however, a plurality of strings 69 of glass fiber or plastics are fixed to the sensor support 26, and when the sensor support 26 is to be lifted, the strings 69 are pulled up to lift the sensor support 26. The lift rod 66 and the strings 69 may be used together.

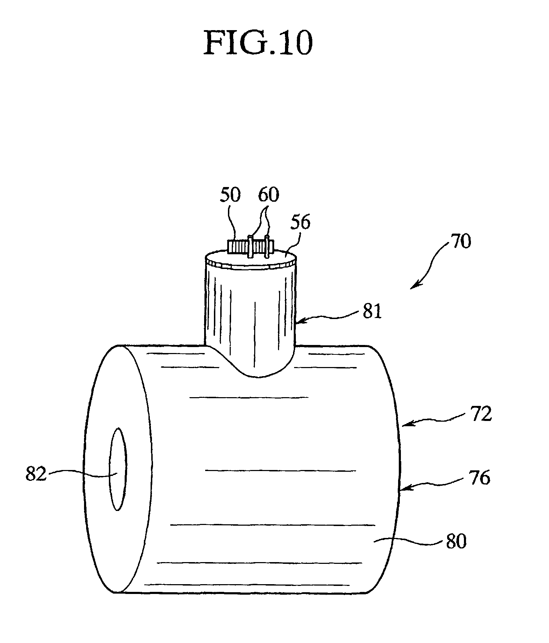

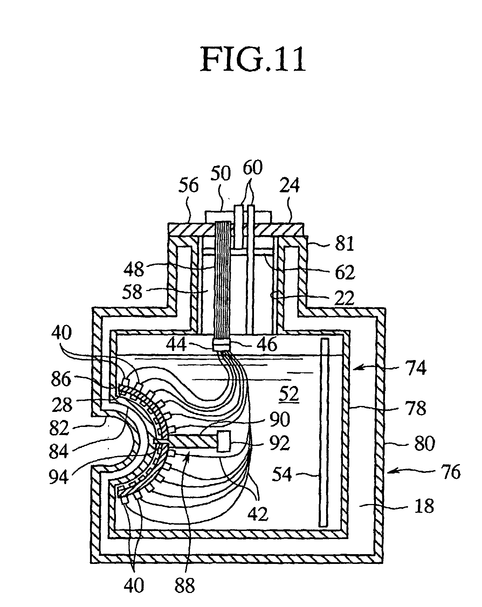

Next, a magnetoencephalographic measuring apparatus according to a second embodiment of the present invention will be explained with reference to FIGS. 10 and 11. FIG. 10 is a perspective view of the measuring apparatus, and FIG. 11 is a vertical sectional view of the measuring apparatus.

As shown in FIGs. 10 and 11, the measuring apparatus 70 has the same structure as the measuring apparatus according to the first embodiment except that the outer vessel 76 includes a concavity 82 in one side.

As shown in FIG 11, the cryostat 72 has a double structure of an inner vessel 74 accommodated in an outer vessel similar to the inner vessel, and a heat insulation material 18 is filled inbetween the inner and the outer vessels 74, 76.

The inner vessel 74 has a cylindrical neck opening 22 projecting upwardly from the substantially central part of an inner vessel body 78. The outer vessel 76 has a cylindrical neck 81 projecting from the substantially central part of an outer vessel body 80. A top opening 24 is in communication with the neck opening 22, and is closed by a cap 56.

As shown in FIG. 11, the helmet-shaped concavity 82 which is opened outward is formed horizontally in one side surface of the outer vessel body 80. The concavity 82 has a helmet-shape and a size which can accommodate the head of a person to be measured. An inner vessel concavity 84 is formed in the inner vessel body 78 in agreement with the concavity 82. A sensor support 86 is provided inside the inner vessel concavity 84. As shown in FIG. 11, the sensor support 86 is formed as a dome of an about 30 cm diameter which covers the inner vessel concavity 84. In the sensor support 86 there is formed a plurality of holes 28 over the entire surface thereof.

On the substantially central part of the surface of the sensor support 86 there is provided a male clamp 88 which is similar to the male clamp 30 of the measuring apparatus 10. The male clamp 88 includes a long handle 90 which reaches substantially to the center of the interior of the inner vessel 74. A sensor 92 which detects environmental magnetic field is mounted on the forward end of the handle 90. One set or a plurality of sets of the sensors 92 may be so mounted. A female clamp 94 which is similar to the female clamp 34 of the measuring apparatus 10 is provided on the inner vessel concavity 84.

Magnetic field sensors 40 comprising SQUIDs are disposed in the respective holes of the sensor support 86. Each of the magnetic field sensors 40 has a rod shape and is inserted in each of the holes to be secured to the sensor support 86.

The respective magnetic field sensors 40 are wired to an output connector 50 through measuring wires 42, a wire connector 44, a plug connector 46 and a wiring loom 48.

At first, liquid helium 52 is charged into the inner vessel body 78 up to an 80%-level so that the magnetic field sensors 40 are immersed. The liquid helium 52 is a coolant for the magnetic field sensors 40. A liquid level of the liquid helium 52 in the inner vessel body 78 can be detected by a level sensor 54 disposed inside the inner vessel body 78.

The measuring wires 42 are formed of copper wires, and have good heat conduction which makes a temperature of the inner vessel body 78 uniform. As a result, even when a liquid level of the liquid helium is lowered, and the magnetic field sensors 40 are exposed to gas phase, temperature increase can be suppressed, and the magnetic field detection is not affected.

Most of the constituent members of the cryostat 72 are formed of non-magnetic and non conducting material, such as fiber reinforced resins, etc. so that they do not interfere with the detection of magnetic fields.

The mounting of the magnetic field sensors 40 on the sensor support 86 is carried out as follows when the measuring apparatus 70 is assembled at the start, and when defective ones of the magnetic field sensors 40 are replaced by new ones for maintenance.

In order to mount the magnetic field sensors 40 on the sensor support 86 having a larger diameter than an inner diameter than the neck opening 22 of the inner vessel 74, as in the above-described measuring apparatus 10, an operator puts his hand into inner vessel 74 through the neck opening 22 in the state where the sensor support 86 is pulled up to the lower end of the neck opening 22.

In the present embodiment, the method for mounting the magnetic field sensors 40 on the sensor support 86 and assembling the measuring apparatus 70 is substantially the same as that for assembling the measuring apparatus according to the first embodiment, but is different from the first embodiment in the method for removing the sensor support 86 from the inner vessel concavity 84.

In removing the sensor support 86, a hand is put into the inner vessel 74 through the neck opening 22 to pull the male clamp 88 and disengage the male clamp 88 from the female clamp 94. The sensor support 86 removed from the inner vessel concavity 84 is rested on a rod support (not shown) through a lift rod or lift strings (not shown).

Thus, without taking the sensor support 86 out of the inner vessel 74, the magnetic field sensors 40 are mounted on the sensor support 86 to assemble the measuring apparatus 70.

Similarly without taking the sensor support 86 out of the inner vessel 74, the magnetic field sensors 40 mounted on the sensor support 86 may be replaced for maintenance.

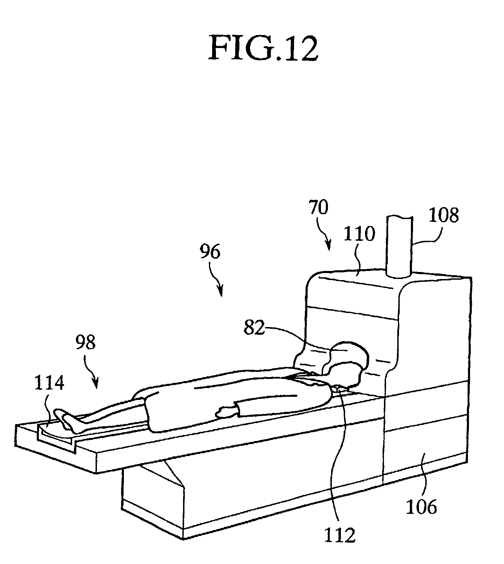

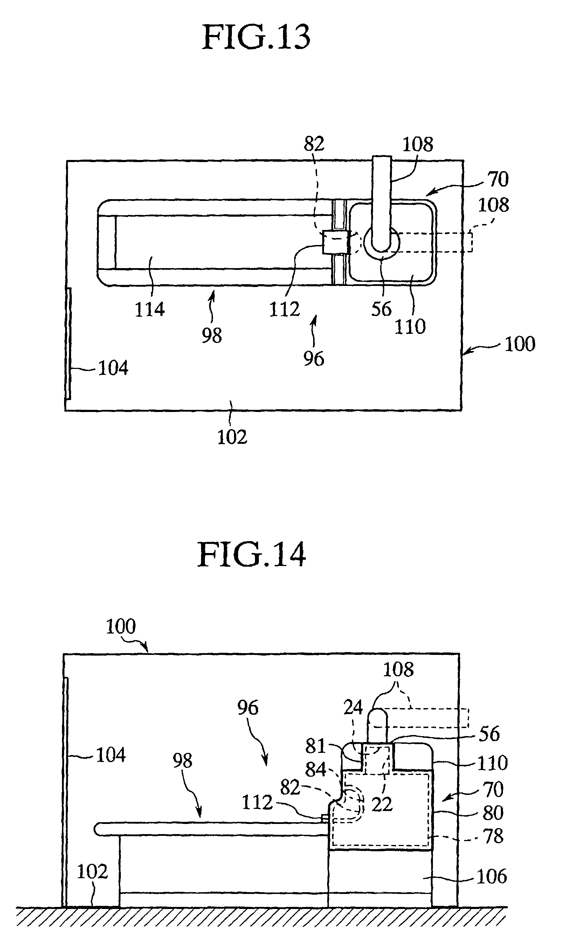

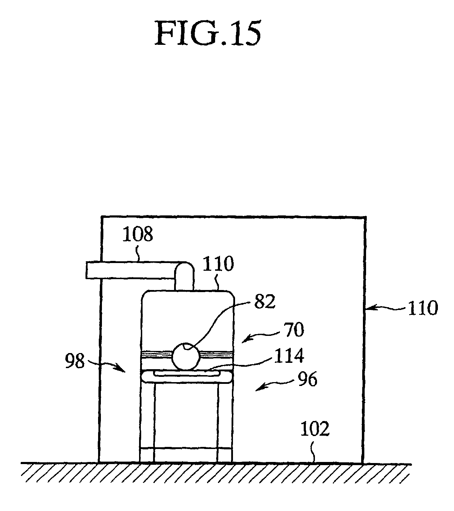

One example of a diagnostic apparatus using the measuring apparatus according to the present embodiment will now be explained with reference to FIGs. 12 to 15. FIG. 12 is a perspective view of the diagnostic apparatus, FIG. 13 is a plan view of the diagnostic apparatus, FIG. 14 is a front view of the diagnostic apparatus, and FIG. 15 is a side view of the diagnostic apparatus.

As shown in FIG. 12, the diagnostic apparatus 96 comprises the measuring apparatus 70 disposed on one end of a bed for a patient to lie on. As shown in FIGs. 13 to 15, the diagnostic apparatus 96 is placed on a floor 102 in a magnetically shielded room 100.

The magnetically shielded room 100 has an entrance/exit door 104. The cryostat 72 of the measuring apparatus 70 is mounted on a table 106. A duct 108 is provided in the top side of the cryostat 72 opposed to the bed and upwardly extended outside. The duct 108 accommodates a charge/discharge pipe 60 provided in the cap 56, and the wiring (not shown) from the output connector 50. The duct 108 may be disposed in another side and extended outside the magnetically shielded room 100 as indicated by the dotted line. The top side of the cryostat 72 except the duct 108 is covered with a cover 110.

The bed 98 is positioned with the height adjusted so that the head of a patient lying on the bed 98 can be inserted into the concavity 82 of the cryostat 72. The bed 98 includes a movable table 114 with a pillow 112 on the side of the concavity 82. The movable table 114 is moved to and from the concavity 82 with a patient lying thereon with the head placed on the pillow 112.

Thus, the patient can have magnetic field distributions occurring in the head detected by the magnetic field sensors 40 while lying on the bed 98.

The measuring apparatus 70 includes the magnetic field sensors 40 arranged so as to cover the head of a patient lying horizontally. Thus the cryostat 72 is lower in height than if the patient were upright, and the measuring apparatus 70 is also lower in height.

Accordingly, the ceiling of the magnetically shielded room 100 for the measuring apparatus 70 can be lowered. As a result, the magnetically shielded room 100 including the diagnostic apparatus can be placed in an ordinary room having low ceilings.

The present invention is not limited to the above-described embodiments and covers other various modifications.

For example, the measuring surface of the sensor support 26, 86 is not essentially disk shaped and helmet-shaped. The cryostat 12, 72 including the inner vessel 14, 74 and the outer vessel 16, 76 are not essentially cylindrical. The neck opening 22 of the inner vessel 14, 74 is not essentially cylindrical.

The magnetic field sensors 40 have been described as being mounted by putting a hand into the neck opening 22, but may be mounted by the use of mounting means which can be operated by remote control.

In engaging and disengaging the sensor support 26, 86 with and from the inner vessel 14, 74, without using a lift rod, an arm may be extended into the inner vessel 14, 74 through the neck opening 22 to directly hold the knob 31. (FIG. 5) or the long handle 90 (FIG. 11) so as to engage and disengage the sensor support 26, 86.

## Claims

1. An apparatus (10 or 70) for measuring weak biomagnetic fields including a plurality of magnetic field sensors (40) and a coolant vessel (12 or 72) having an interior with a measuring surface, said measuring surface being an interior surface of said coolant vessel, and a neck opening (22),
**characterized in that** the apparatus comprises:
a sensor support (26 or 86) for disengageably supporting said plurality of magnetic field sensors (40), said sensor support (26 or 86) being disposed in said interior at said measuring surface; and
fixing means (30, 31, 32, 34, 36 or 88, 90, 92, 94) for releasably fixing said sensor support (26 or 86), with said plurality of magnetic field sensors (40) mounted thereon, to said measuring surface of the coolant vessel (12 or 72), the releasability of the fixing means permitting removal of said sensor support (26 or 86) from said measuring surface;
wherein said neck opening (22) provides access to said plurality of magnetic field sensors (40) and has a diameter smaller than that of the sensor support (26 or 86) and greater than that of individual magnetic field sensors (40) to permit installation of said magnetic field sensors (40) on said sensor support (26 or 86) via said neck opening (22).

2. An apparatus (70) according to claim 1, wherein:
said measuring surface of said coolant vessel (72) is formed in a side of said coolant vessel (72) and follows the shape of a concavity in an outside surface of said coolant vessel (72), which concavity is adapted to cover at least a part of a head of a human being; and
said sensor support (86) has a configuration which permits said magnetic field sensors to be disposed in conformance with a contour of the measuring surface.

3. An assembly method for assembling an apparatus (10 or 70) according to claim 1 or 2, comprising the steps of:
releasing said sensor support (26 or 86) from said measuring surface of said coolant vessel (12 or 72);
holding said sensor support (26 or 86) within said coolant vessel (12 or 72) near to said opening (22);
mounting said magnetic field sensors (40) on said sensor support (26 or 86); and
releasably fixing said sensor support (26 or 86) with said magnetic field sensors (40) mounted thereon to said measuring surface of said coolant vessel (12 or 72).

4. A maintenance method for maintaining an apparatus (10 or 70) according to claim 1 or 2, comprising the steps of:
disengaging said sensor support (26 or 86) from said measuring surface of said coolant vessel (12 or 72);
holding said sensor support (26 or 86) within said coolant vessel (12 or 72) near to said opening (22);
removing a defective magnetic field sensor (40) from the sensor support (26 or 86);
mounting a normal magnetic field sensor (40) in place of said defective magnetic field sensor (40) on said sensor support (26 or 86); and
releasably fixing said sensor support (26 or 86) with said magnetic field sensors (40) mounted thereon to said measuring surface of said coolant vessel (12 or 72).

5. A diagnostic apparatus for measuring weak biomagnetic fields, comprising:
a bed (98) for a patient to be diagnosed to lie on;
an apparatus (70) according to claim 2 disposed on an end of the bed (98) and arranged so that the head of the patient can be positioned in the concavity near to the measuring surface.

## Patentansprüche

1. Vorrichtung (10 oder 70) zum Messen schwacher biomagnetischer Felder, die eine Vielzahl magnetischer Feldsensoren (40) und einen Kühlmittelbehälter (12 oder 72) aufweist, der einen Innenraum mit einer Meßoberfläche hat, wobei die Meßoberfläche eine innere Oberfläche des Kühlmittelbehälters ist, sowie eine Halsöffnung (22),
**dadurch gekennzeichnet, daß** die Vorrichtung aufweist:
einen Sensorträger (26 oder 86) zum lösbaren Halten der Vielzahl der magnetischen Feldsensoren (40), wobei der Sensorträger (26 oder 86) in dem Innenraum an der Meßoberfläche angeordnet ist; und
Befestigungsmittel (30, 31, 32, 34, 36 oder 88, 90, 92, 94) zum lösbaren Befestigen des Sensorträgers (26 oder 86), an dem die Vielzahl der magnetischen Feldsensoren (40) angebracht ist, an der Meßoberfläche des Kühlmittelbehälters (12 oder 72), wobei die Lösbarkeit der Befestigungsmittel eine Entfernung des Sensorträgers (26 oder 86) von der Meßoberfläche gestattet;
wobei die Halsöffnung (22) einen Zugang zu der Vielzahl der magnetischen Feldsensoren (40) ermöglicht und einen Durchmesser hat, der kleiner ist als derjenige des Sensorträgers (26 oder 86) und größer als derjenige individueller magnetischer Feldsensoren (40), um eine Installation der magnetischen Feldsensoren (40) an dem Sensorträger (26 oder 86) über die Halsöffnung (22) zu gestatten.

2. Vorrichtung (70) nach Anspruch 1, wobei:
die Meßoberfläche des Kühlmittelbehälters (72) in einer Seite des Kühlmittelbehälters (72) ausgebildet ist und der Form einer Wölbung in einer äußeren Oberfläche des Kühlmittelbehälters (72) folgt,wobei die Wölbung so ausgelegt ist, daß sie mindestens einen Teil eines Kopfes eines Menschen bedeckt; und
wobei der Sensorträger (86) eine Konfiguration hat, die es gestattet, daß die magnetischen Feldsensoren in Übereinstimmung mit einer Kontur der Meßoberfläche angeordnet sind.

3. Montageverfahren zum Aufbauen einer Vorrichtung (10 oder 70) nach Anspruch 1 oder 2, das die folgenden Schritte aufweist:
Lösen des Sensorträgers (26 oder 86) von der Meßoberfläche des Kühlmittelbehälters (12 oder 72);
Halten des Sensorträgers (26 oder 86) innerhalb des Kühlmittelbehälters (12 oder 72) in der Nähe der Öffnung (22);
Anbringen der magnetischen Feldsensoren (40) an dem Sensorträger (26 oder 86); und
lösbares Befestigen des Sensorträgers (26 oder 86), an dem die magnetischen Feldsensoren (40) angebracht sind, an der Meßoberfläche des Kühlmittelbehälters (12 oder 72).

4. Wartungsverfahren zum Instandhalten einer Vorrichtung (10 oder 70) nach Anspruch 1 oder 2, das die folgenden Schritte aufweist:
Lösen des Sensorträgers (26 oder 86) von der Meßoberfläche des Kühlmittelbehälters (12 oder 72);
Halten des Sensorträgers (26 oder 86) innerhalb des Kühlmittelbehälters (12 oder 72) in der Nähe der Öffnung (22);
Entfernen eines defekten magnetischen Feldsensors (40) von dem Sensorträger (26 oder 86);
Anbringen eines normalen magnetischen Feldsensors (40) anstelle des defekten magnetischen Feldsensors (40) an dem Sensorträger (26 oder 86); und
lösbares Befestigen des Sensorträgers (26 oder 86), an dem die magnetischen Feldsensoren (40) angebracht sind, an der Meßoberfläche des Kühlmittelbehälters (12 oder 72).

5. Diagnosevorrichtung zum Messen schwacher biomagnetischer Felder, aufweisend:
ein Bett (98), auf dem ein zu untersuchender Patient liegen kann;
Vorrichtung (70) nach Anspruch 2, die an einem Ende des Betts (98) angeordnet und so eingerichtet ist, daß der Kopf des Patienten in der Wölbung in der Nähe der Meßoberfläche positioniert werden kann.

## Revendications

1. Appareil (10 ou 70) destiné à mesurer les champs biomagnétiques faibles y compris une pluralité de capteurs de champ magnétique (40) et une cuve de refroidissement (12 ou 72) ayant un intérieur muni d'une surface de mesure, ladite surface de mesure étant une surface intérieure de ladite cuve de refroidissement, et ayant une ouverture de type goulot (22),
**caractérisé en ce que** l'appareil comporte
un support de capteurs (26 ou 86) destiné à supporter de manière débrayable ladite pluralité de capteurs de champ magnétique (40), ledit support de capteurs (26 ou 86) étant disposé dans ledit intérieur sur ladite surface de mesure ; et
des moyens de fixation (30, 31, 32, 34, 36 ou 88, 90, 92, 94) destinés à fixer de manière libérable ledit support de capteurs (26 ou 86), ladite pluralité de capteurs de champ magnétique (40) y étant montée, sur ladite surface de mesure de la cuve de refroidissement (12 ou 72), l'aspect libérable des moyens de fixation permettant la dépose dudit support de capteurs (26 ou 86) de ladite surface de mesure ;
dans lequel ladite ouverture de type goulot (22) procure un accès à ladite pluralité de capteurs de champ magnétique (40) et a un diamètre inférieur à celui du support de capteurs (26 ou 86) et supérieur à celui de chacun des capteurs de champ magnétique (40) afin de permettre l'installation desdits capteurs de champ magnétique (40) sur ledit support de capteurs (26 ou 86) par le biais de l'ouverture de type goulot (22).

2. Appareil (70), suivant la revendication 1, dans lequel :
ladite surface de mesure de ladite cuve de refroidissement (72) est formée dans un côté de ladite cuve de refroidissement (72) et prend la forme d'une concavité sur une surface extérieure de ladite cuve de refroidissement (72), concavité qui est adaptée pour couvrir au moins une partie d'une tête d'un être humain ; et
ledit support de capteurs (86) présente une configuration qui permet aux capteurs de champ magnétique d'être disposés en conformité avec un contour de la surface de mesure.

3. Procédé d'assemblage pour assembler un appareil (10 ou 70), suivant la revendication 1 ou la revendication 2, comportant les étapes suivantes :
la libération dudit support de capteurs (26 ou 86) de ladite surface de mesure de ladite cuve de refroidissement (12 ou 72) ;
le maintien dudit support de capteurs (26 ou 86) dans ladite cuve de refroidissement (12 ou 72) à proximité de ladite ouverture (22) ;
le montage desdits capteurs de champ magnétique (40) sur ledit support de capteurs (26 ou 86) ; et
de manière libérable la fixation dudit support de capteurs (26 ou 86), muni desdits capteurs de champ magnétique (40) qui y sont montés, sur ladite surface de mesure de ladite cuve de refroidissement (12 ou 72).

4. Procédé de maintenance pour maintenir un appareil (10 ou 70) suivant la revendication 1 ou la revendication 2, comportant les étapes suivantes :
le débrayage dudit support de capteurs (26 ou 86) de ladite surface de mesure de ladite cuve de refroidissement (12 ou 72) ;
le maintien dudit support de capteurs (26 ou 86) dans ladite cuve de refroidissement (12 ou 72) à proximité de ladite ouverture (22) ;
la dépose d'un capteur de champ magnétique (40) défectueux dudit support de capteurs (26 ou 86) ;
le montage d'un capteur de champ magnétique (40) normal à la place dudit capteur de champ magnétique (40) défectueux sur ledit support de capteurs (26 ou 86) ; et
de manière libérable la fixation dudit support de capteurs (26 ou 86), muni desdits capteurs de champ magnétique (40) qui y sont montés, sur ladite surface de mesure de ladite cuve de refroidissement (12 ou 72).

5. Appareil de diagnostic destiné à mesurer les champs biomagnétiques faibles, comportant
un lit (98) pour qu'un patient devant être diagnostiqué s'y allonge ;
un appareil (70), suivant la revendication 2, disposé à une extrémité du lit (98) et disposé de telle manière que la tête du patient puisse être placée dans la concavité à proximité de la surface de mesure.
